(19) Europäisches Patentamt
European Patent Office
Office européen des brevets

(11) Publication number: 0 100 915
A2

# (12) EUROPEAN PATENT APPLICATION

(21) Application number: 83106963.8

(22) Date of filing: 15.07.83

(51) Int. Cl.[3]: A 61 K 7/06
A 61 K 7/48

(30) Priority: 15.07.82 JP 123527/82

(43) Date of publication of application:
22.02.84 Bulletin 84/8

(84) Designated Contracting States:
AT BE CH DE FR GB IT LI NL SE

(71) Applicant: Eisai Co., Ltd.
6-10, Koishikawa 4-chome Bunkyo-ku
Tokyo 112(JP)

(72) Inventor: Hirama, Shinichi
Niiha-Ryo 338, Niiha-cho Kohoku-ku
Yokohama-shi Kanagawa Prefecture(JP)

(72) Inventor: Nishiyama, Toshio
3-13-6-806, Minami-Rokugo
Ohta-ku Tokyo(JP)

(72) Inventor: Ohta, Youichi
26, Nobi Yokosuka-shi
Kanagawa Prefecture(JP)

(72) Inventor: Uzuka, Makoto
1350-3, Nakada-cho Totsuka-ku
Yokohama-shi Kanagawa Prefecture(JP)

(72) Inventor: Tomita, Kenichi
15-9, Iwabuchi-cho
Kita-ku Tokyo(JP)

(72) Inventor: Nakajima, Keisuke
2-26-16, Moriga-oka Isogo-ku
Yokohama-shi Kanagawa Prefecture(JP)

(72) Inventor: Furuse, Kazumaro
3-5, Inogashira 2-chome
Mitaka-shi Tokyo(JP)

(74) Representative: Hansen, Bernd, Dr.rer.nat. et al,
Hoffmann, Eitle & Partner Patentanwälte
Arabellastrasse 4
D-8000 München 81(DE)

## (54) Hair grower composition.

(57) A hair grower composition comprising a ubiquinone (coenzyme $Q_n$) represented by the general formula:

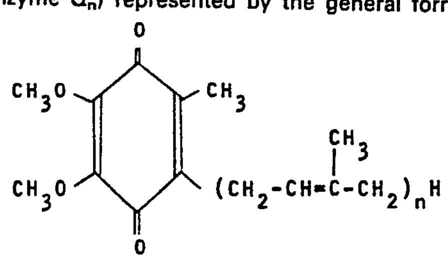

wherein n represents an integer of from 7 to 10. In addition to such a ubiquinone, the hair grower composition may contain therein a skin peripheral vasolidator drug, such as carpronium chloride, vitamin E nicotinate and benzyl nicotinate.

EP 0 100 915 A2

Hair Grower Composition

The present invention relates to a hair grower composition.

Ubiquinones have been discovered from the mitochondria lipid of the heart of bovines by F.L. Crane in 1957. Presently, some ubiquinones are known, and they are only different in the number (n) of isoprenoid side chains from one another. Their action *in vivo* is not well known, but it is considered that they play a very important role in an electrone transport system to supplying energy required for actions of cells.

The inventors have discovered that ubiquinone-containing drugs exhibit a hair growing effect, and also the fact that a ubiquinone alone has a hair growing effect, and ubiquinone combined with a skin peripheral vasodilator drug, such as carpronium chloride, vitamin E nicotinate, benzyl nicotinate, exhibits an even more excellent hair growing effect, and they have thus accomplished the present invention.

It is therefore an object of the present invention to provide a hair grower composition, which comprises either a ubichinone (coenzyme $Q_n$) presented by the general furmula:

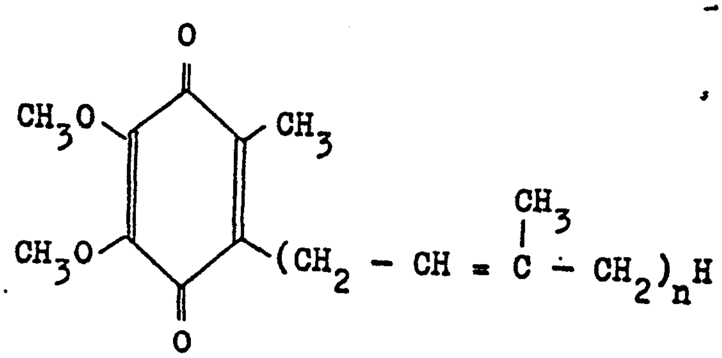

wherein n represents an integer of from 7 to 10, or such a ubiquinone and a skin peripheral vasodilator drug.

The present invention also comprises a method for the preparation of a hair grower composition by admixing the above-mentioned compound(s) with components usually contained in hair grower compositions.

The present invention will now be described in detail with respect to the features thereof. Ubiquinones used in the present invention are those having 7 to 10 isoprenoid side chains, and among them, ubiquinone having 10 isoprenoid side chains (which will be referred to as ubiquinone-10 hereinafter) is particularly excellent. Ubiquinones may be incorporated, preferably in an amount within a range of 0.01 to 2 % by weight.

Skin peripheral vasodilator drugs used in this invention are carpronium chloride, vitamin E nicotinates, or benzyl nicotinates, and one or more of them can be optionally selected. They may be preferably incorporated in an amount within a range of 0.1 to 2 % by weight.

The hair grower composition according to the present invention has an excellent hair growing effect, and also may cause no problem with respect to safety.

The present invention will now be more particularly described by the following tests and examples, but it should be noted that the latter are not intended in any way to limit the scope of the invention. All amounts of materials added are by weight.

Test

The following efficiency test was carried out for the purpose of demonstrating an excellent hair growing effect of a hair grower composition according to the present invention.

(1) Three samples as given below were used.

(a)

| Hair grower composition | % by weight |
|---|---|
| 95 % ethanol | 50 |
| Ubiquinone-10 | 0.5 |
| Panthotenyl ethyl ether | 0.1 |
| Hardened caster oil-40 mole ethylene oxide adduct | 2 |
| Ion-exchanged water | 47.4 |

A perfume and a coloring agent were incorporated in an appropriate amount.

(b)

| Hair grower composition | % by weight |
|---|---|
| 95 % ethanol | 50 |
| Carpronium chloride | 1 |
| Panthotenyl ethyl ether | 0.1 |
| Hardened caster oil-40 mole ethylene oxide adduct | 2 |
| Ion-exchanged water | 46.9 |

A perfume and a coloring agent were incorporated in an appropriate amount.

| (c) Hair tonic composition | % by weight |
|---|---|
| 95 % ethanol | 50 |
| Ubiquinone-10 | 0.5 |
| Carpronium chloride | 1. |
| Panthotenyl ethyl ether | 0.1 |
| Hardened caster oil-40 mole ethylene oxide adduct | 2 |
| Ion-exchanged water | 46.4 |

A perfume and a coloring agent were incorporated in an appropriate amount.

(2) In this test, the effect was evaluted in dependence on the number of hairs fallen out at washing of the hair before and after the hair growers were used. Panels to be subjected to the test were ten for each of the above-mentioned samples. Hairs fallen out were collected once a week: 16 times in total for a period of four months. This period of four months was divided into the following two periods: a period of initial two months for collecting hairs naturally fallen out, and a period of the later two months for collecting hair fallen out after application of the above samples to examine the effects upon the application thereof. The respective effect was evaluated by comparing the average number of fallen out hairs which were collected eight times for a period before use of the hair growers (which will be referred to as a non-applied period hereinafter) with that of fallen out hairs which were similarly collected eight times for a period after

application of the hair growers (which will be referred to as an applied period). The hairs were washed at intervals of every two days.

(3) Effect

As shown in Table I, with sample (a), in which ubiquinone-10 is incorporated, the following data were obtained: among ten panels, a remarkably good effect (++) was shown in one panel; a good effect (+) in five panels; a slightly good effect (±) in two panels; and no effect (-) in two panels. This means that ubiquinone-10 has an excellent hair grower effect. The result is given in Table I.

With sample (b), in which carpronium chloride is incorporated but ubiquinone is not incorporated, the above-given effects were not found, i.e. the following result was obtained: among ten panels, a remarkably good effect (++) was shown in non of the panels; a good effect (+) in two panels; a slightly good effect (±) in four panels; and no effect (-) in four panels. The result is given in Table II.

With sample (c), in which ubiquinone-10 and carpronium chloride are incorporated, the following result was obtained: among ten panels, a remarkably good effect (++) was shown in five panels; a good effect (+) in three panels; a slightly good effect (±) in tow panels. This means that while no effect is shown with skin peripheral vasodilator drugs, the addition addition of ubiquinone-10 thereto increases the effect of ubiquinone-10 in a synergistic way. The result is given in Table III. Furthermore, the criteria in evaluation of effects in the tables are as follows:

| Reduced number of hairs fallen out | |
|---|---|
| more than 70 | remarkably good effect (++) |
| more than 30 and less than 70 | good effect (+) |
| more than 10 and less than 30 | slightly good effect (±) |
| less than 10 | no effect (-) |

Example 1

| Hair grower composition (of a lotion type) | % by weight |
|---|---|
| 95 % ethanol | 85 |
| Ubiquinone-10 | 0.01 |
| Biotin | 0.01 |
| Hinokitiol | 0.01 |
| ℓ-menthol | 0.2 |
| Ion-exchanged water | 14.77 |

In addition, a perfume and a coloring agent are incorporated in an appropriate amount.

Preparation --- ubiquinone-10, biotin, hinokitiol, ℓ-menthol, a perfume and a coloring agent are added to ethanol and dissolved therein by stirring, followed by adding ion-exchanged water to mix them, thereby to provide a clear liquid hair grower composition.

Example 2

| Hair grower composition (of a lotion type) | % by weight |
|---|---|
| 95 % ethanol | 50 |
| Ubiquinone-10 | 0.7 |

| | |
|---|---|
| Ubiquinone-7 | 0.3 |
| Carpronium chloride | 0.5 |
| Panthotenyl ethyl ether | 0.1 |
| Hardened caster oil-40 mole ethylene oxide adduct | 20 |
| Ion-exchanged water | 46.4 |

In addition, a perfume and a coloring agent are incorporated in an appropriate amount, respectively.

Preparation --- the procedure was repeated as in Example 1, except that components were used in the amounts indicated above.

Example 3

| Hair grower composition (of a cream type) | | % by weight |
|---|---|---|
| A: | Liquid paraffin | 38 |
| | Stearic acid | 2 |
| | White vaseline | 10 |
| | Ubiquinone-10 | 1 |
| | Vitamin E nicotinate | 0.1 |
| | Polyoxyethylene sorbitan mono-stearate | 3.8 |
| | Sorbitan mono-stearate | 4.2 |
| | Ethyl paraben | trace |
| B: | Triethanolamine | 1 |
| | Polyethylene glycol 1500 | 2 |
| | Ion-exchanged water | 37.9 |
| | Perfume | appropriate amount |

Preparation --- the above oil phase (A) and aqueous phase (B) were heated to a temperature of 80°C, respectively, and mixed together for emulsification at the same temperature, followed by cooling the emulsified mass to an ambient temperature with stirring, thereby to provide a hair grower composition.

Example 4

| | Hair grower composition (of a milky lotion type) | % by weight |
|---|---|---|
| A: | Purified jojoba oil | 10 |
| | Ubiquinone-10 | 2 |
| | Panthotenyl ethyl ether | 0.01 |
| | Benzyl nicotinate | 2 |
| | Lanolin | 2 |
| | Iso-propyl myristate | 2.5 |
| | Polyoxyethylene cetyl alcohol | 1.8 |
| | Sorbitan mono-stearate | 0.8 |
| | Ethyl paraben | trace |
| B: | Triethanolamine | 1 |
| | Glycerine | 4 |
| | Ion-exchanged water | 73.89 |
| | Perfume | appropriate amount |

Preparation --- the above oil phase (A) and aqueous phase (B) were heated to a temperature of 80°C, respectively, and mixed together for emulsification at the same temperature, followed by cooling the emulsified mass to an

ambient temperature with stirring, thereby to provide a hair grower composition.

 

Table I

| Panel No. | Non-applied period | Applied period | Effect |
|---|---|---|---|
| 1 | 226 ± 51 | 203 ± 37 | ± |
| 2 | 193 ± 33 | 150 ± 29 | + |
| 3 | 343 ± 34 | 291 ± 61 | + |
| 4 | 234 ± 28 | 196 ± 31 | + |
| 5 | 156 ± 29 | 171 ± 41 | - |
| 6 | 369 ± 81 | 281 ± 74 | ++ |
| 7 | 403 ± 61 | 351 ± 45 | + |
| 8 | 311 ± 75 | 289 ± 61 | ± |
| 9 | 231 ± 29 | 179 ± 44 | + |
| 10 | 199 ± 61 | 196 ± 49 | - |

Table II

| Panel No. | Non-applied period | Applied period | Effect |
|---|---|---|---|
| 11 | 231 ± 61 | 232 ± 48 | - |
| 12 | 310 ± 89 | 303 ± 71 | - |
| 13 | 249 ± 54 | 226 ± 63 | ± |
| 14 | 199 ± 29 | 151 ± 28 | + |
| 15 | 333 ± 70 | 339 ± 61 | - |
| 16 | 401 ± 51 | 363 ± 49 | + |
| 17 | 267 ± 29 | 239 ± 51 | ± |
| 18 | 181 ± 38 | 163 ± 29 | ± |
| 19 | 280 ± 49 | 282 ± 61 | - |
| 20 | 232 ± 63 | 210 ± 56 | ± |

Table III

| Panel No. | Non-applied period | Applied period | Effect |
|---|---|---|---|
| 11 | 345 ± 81 | 260 ± 72 | ++ |
| 12 | 299 ± 71 | 212 ± 39 | ++ |
| 13 | 197 ± 29 | 151 ± 37 | + |
| 14 | 331 ± 61 | 283 ± 32 | + |
| 15 | 431 ± 101 | 403 ± 111 | ± |
| 16 | 312 ± 81 | 230 ± 62 | ++ |
| 17 | 228 ± 51 | 153 ± 42 | ++ |
| 18 | 233 ± 39 | 191 ± 27 | + |
| 19 | 342 ± 60 | 258 ± 44 | ++ |
| 20 | 293 ± 51 | 261 ± 70 | ± |

CLAIMS

1. A hair grower composition which comprises a ubiquinone (coenzyme $Q_n$) represented by the general formula:

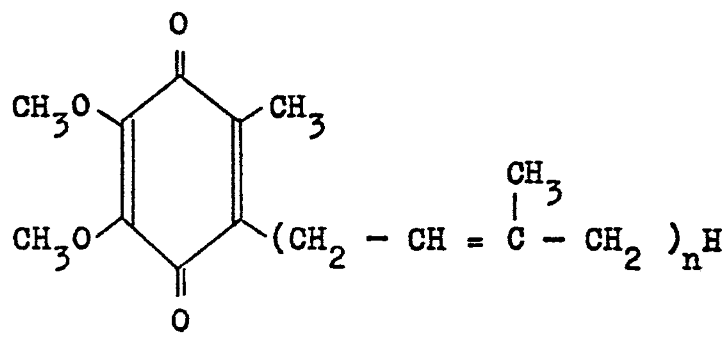

wherein n represents an integer of from 7 to 10.

2. A hair grower composition according to claim 1, wherein the ubiquinone is ubiquinone-10.

3. A hair grower composition which comprises a ubiquinone (coenzyme $Q_n$) represented by the general formula:

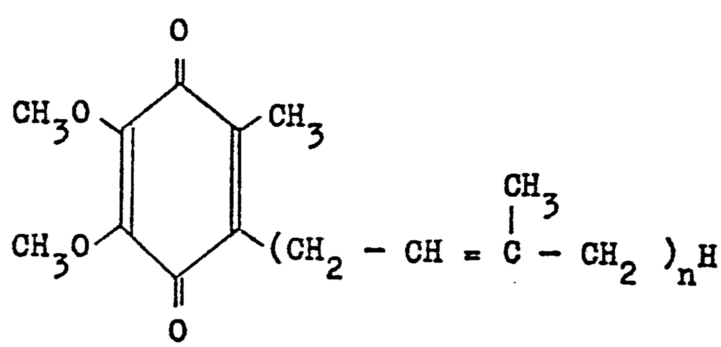

wherein n represents an integer of from 7 to 10, and a skin peripheral vasodilator drug.

4. A hair grower composition according to claim 3, wherein the skin peripheral vasodilator drug is one or more compounds selected from carpronium chloride, vitamin E nicotinate and benzyl nicotinate.

5. A hair grower composition according to claim 3 or 4, wherein the ubiquinone is ubiquinone-10.

1. A hair grower composition which comprises a ubiquinone (coenzyme $Q_n$) represented by the general formula:

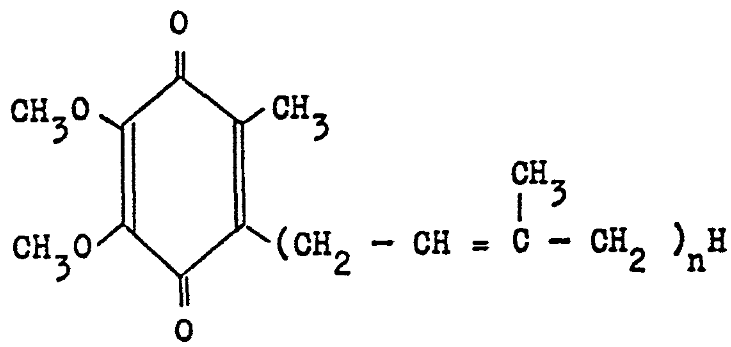

wherein n represents an integer of from 7 to 10.

2. A hair grower composition according to claim 1, wherein the ubiquinone is ubiquinone-10.

3. A hair grower composition which comprises a ubiquinone (coenzyme $Q_n$) represented by the general formula:

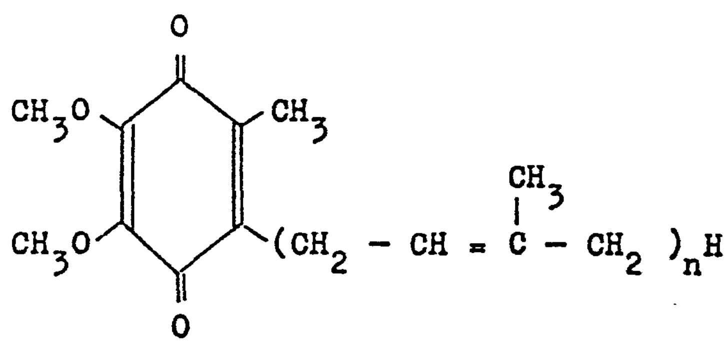

wherein n represents an integer of from 7 to 10 , and a skin peripheral vasodilator drug.

4. A hair grower composition according to claim 3, wherein the skin peripheral vasodilator drug is one or more compounds selected from carpronium chloride, vitamin E nicotinate and benzyl nicotinate.

5. A hair grower composition according to claim 3 or 4, wherein the ubiquinone is ubiquinone-10.

6. A method for the preparation of a hair grower composition, characterized by dissolving or dispersing a ubiquinone (coenzyme $Q_n$) represented by the general formula

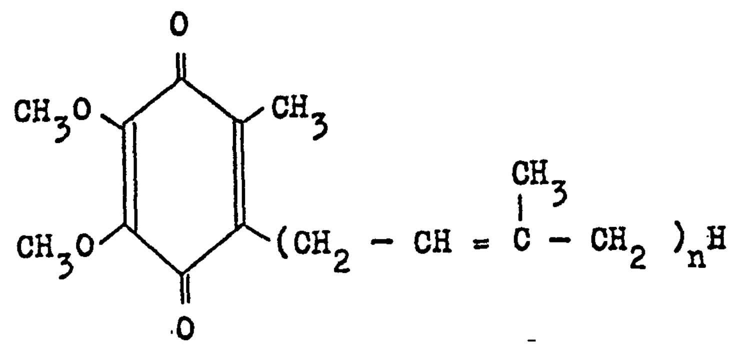

wherein n represents an integer of from 7 to 10, in a suitable dissolving or dispersing medium.

7. A method according to claim 6, characterized in that as ubiquinone ubiquinone-10 is chosen.

8. A method for the preparation of a hair grower composition, characterized by dissolving or admixing a ubiquinone (coenzyme $Q_n$) represented by the general formula:

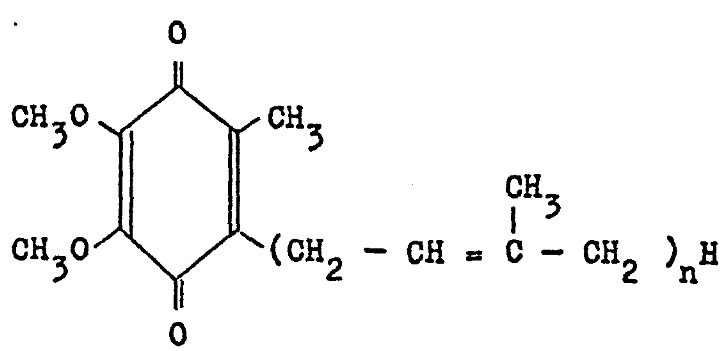

Austria

wherein n represents an integer of from 7 to 10, and a skin peripheral vasodilator drug, with a suitable dissolving or despersing medium.

9. A method according to claim 8, characterized in that the skin peripheral vasodilator drug is chosen from the group of carpronium chloride, vitamin E nicotinate and benzyl nicotinate.

10. A method according to claim 8, characterized in that as ubiquinone ubiquinone-10 is chosen.